(19) Europäisches Patentamt
European Patent Office
Office européen des brevets

(11) Publication number: 0 126 230 A1

# (12) EUROPEAN PATENT APPLICATION

(21) Application number: 84102775.8

(22) Date of filing: 14.03.84

(51) Int. Cl.³: C 12 N 15/00, C 12 N 1/20 // (C12N1/20, C12R1/19)

(30) Priority: 17.03.83 JP 45723/83

(43) Date of publication of application: 28.11.84 Bulletin 84/48

(84) Designated Contracting States: AT BE CH DE FR GB IT LI LU NL SE

(71) Applicant: Takeda Chemical Industries, Ltd., 27, Doshomachi 2-chome Higashi-ku, Osaka-shi Osaka, 541 (JP)

(72) Inventor: Kikuchi, Masakazu, 4-16, Higashitokiwadai 7-chome Toyono-cho, Toyono-gun Osaka 563-1 (JP)
Inventor: Tsukamoto, Kyozo, A-204, 39 Sneriyamanishi 4-chome, Suita Osaka 565 (JP)
Inventor: Kurokawa, Tsutomu, 1-50, Suimeidai 1-chome, Kawanishi Hyogo 666-01 (JP)

(74) Representative: von Kreisler, Alek, Dipl.-Chem. et al, Deichmannhaus am Hauptbahnhof, D-5000 Köln 1 (DE)

## (54) Novel DNA and use thereof.

(57) Human immune interferon protein can be obtained by growing a transformant containing a recombinant DNA having a structural gene of human immune interferon downstream from a rec A promoter or promoters and recovering the human immune interferon protein accumulated in the culture broth.

EP 0 126 230 A1

# Novel DNA and Use Thereof

This invention relates to a recombinant DNA and use thereof.

More particularly, this invention relates to a recombinant DNA containing the structural gene for human immune interferon downstream from a rec A promoter or promoters and use hereof.

Interferons (hereinafter sometimes abbreviated as 'IFN') are proteins produced by cells of higher animals in response to the stimulation with a virus, nucleic acid, etc., and have antiviral, antitumor and other activities.

Three types of human IFN are now known, namely alpha, beta and gamma types (hereinafter sometimes abbreviated as 'IFN-α', 'IFN-β' and 'IFN-γ', respectively). The alpha and beta types are induced by a virus or nucleic acid and the gamma type is induced by mitogens, among others, IFN-γ is also called immune interferon (I-IFN) and said to have higher antiproliferative and antitumor activities as compared with IFN-α and IFN-β and therefore is more promising from the viewpoint of clinical application.

Recently, by making use of gene manipulation techniques, cDNAs complementary to mRNAs isolated from human IFN-γ-secreting cells such as peripheral blood lymphocytes were synthesized. Such human IFN-γ-encoding cDNAs were cloned, their base sequences were determined and the amino acid sequence of human IFN-γ was estimated on the basis of said base sequences. It was thus revealed that the human

IFN-γ gene is composed of a region for a signal peptide containing 20 amino acids and a region coding for the IFN-polypeptide containing 146 amino acids [Gray, P. W. et al., Nature, 295, 503-509 (1982); Devos, R. et al., Nucleic Acids Research, 10, 2487-2501 (1982); Derynck, R. et al., Nucleic Acids Research, 10, 3605-3615 (1982)]. The results of these studies indicate that there are two structural genes for IFN-γ, one for the IFN-γ polypeptide containing Arg as the 140th amino acid (Devos et al., vide supra) and the other for the IFN-γ polypeptide containing Gln as the 140th amino acid (Gray et al. and Derynck et al., vide supra).

Furthermore, a DNA equivalent in gene function to the structural gene for the IFN-γ polypeptide (the 140th amino acid being Arg) was prepared by chemical synthesis [Tanaka, S. et al., Nucleic Acids Research Symposium Series No. 11, 29-32 (1982)]. The expression of these genes was attempted successfully in monkey cells (Gray et al. and Devos et al., vide supra), E. coli [Gray et al., vide supra; Goeddel, D., Abstracts of 4th International Symposium on Genetics of Industrial Microorganisms (Kyoto), p. 30 (1982); Tanaka et al., vide supra] and yeast [Goeddel, vide supra]. However, in each case, the productivity is not so high as to enable large-scale production.

The present inventors energetically endeavored to realize efficient expression of the IFN-γ gene in Escherichia coli and found that said gene can be expressed efficiently when a rec A promoter is utilized, and continued research has now led to the present invention. Thus, the present invention provides a novel method of producing human IFN-γ utilizing a rec A promoter or promoters.

The DNA to be used in the present invention may be any human IFN-γ polypeptide-encoding one, for instance a complementary DNA (cDNA) synthesized on the basis of mRNA, a genomic DNA, a chemically synthesized DNA or a DNA

constructed by adequately combining these. More specifically, there may be mentioned a DNA isolated and identified by the present inventors and containing the base sequence of the formula

```
     1
(5') TGT TAC TGC CAG GAC CCA TAT GTA AAA GAA GCA GAA AAC
CTT AAG AAA TAT TTT AAT GCA GGT CAT TCA GAT GTA GCG GAT
AAT GGA ACT CTT TTC TTA GGC ATT TTG AAG AAT TGG AAA GAG
GAG AGT GAC AGA AAA ATA ATG CAG AGC CAA ATT GTC TCC TTT
TAC TTC AAA CTT TTT AAA AAC TTT AAA GAT GAC CAG AGC ATC
CAA AAG AGT GTG GAG ACC ATC AAG GAA GAC ATG AAT GTC AAG
TTT TTC AAT AGC AAC AAA AAG AAA CGA GAT GAC TTC GAA AAG
CTG ACT AAT TAT TCG GTA ACT GAC TTG AAT GTC CAA CGC AAA
GCA ATA CAT GAA CTC ATC CAA GTG ATG GCT GAA CTG TCG CCA
GCA GCT AAA ACA GGG AAG CGA AAA AGG AGT CAG ATG CTG TTT
140                     146
CGA GGT CGA AGA GCA TCC CAG-X(3')               (I)
```

wherein X is TAA, TGA or TAG [refer to European Patent Publication No. 0089676 claiming the priority of PCT/JP82/00080]. The DNA represented by formula (I) codes for the polypeptide of the formula

```
(N) Cys Tyr Cys Gln Asp Pro Tyr Val Lys Glu Ala Glu Asn
Leu Lys Lys Tyr Phe Asn Ala Gly His Ser Asp Val Ala Asp
Asn Gly Thr Leu Phe Leu Gly Ile Leu Lys Asn Trp Lys Glu
Glu Ser Asp Arg Lys Ile Met Gln Ser Gln Ile Val Ser Phe
Tyr Phe Lys Leu Phe Lys Asn Phe Lys Asp Asp Gln Ser Ile
Gln Lys Ser Val Glu Thr Ile Lys Glu Asp Met Asn Val Lys
Phe Phe Asn Ser Asn Lys Lys Lys Arg Asp Asp Phe Glu Lys
Leu Thr Asn Tyr Ser Val Thr Asp Leu Asn Val Gln Arg Lys
Ala Ile His Glu Leu Ile Gln Val Met Ala Glu Leu Ser Pro
Ala Ala Lys Thr Gly Lys Arg Lys Arg Ser Gln Met Leu Phe
Arg Gly Arg Arg Ala Ser Gln (C)                 (II)
```

or a polypeptide equivalent thereto in immunological or biological activities.

The above DNA (I) may have ATG (III) at the 5'-end thereof. When the DNA (I) has ATG of formula (III) at the 5'-end thereof, it codes not only for the polypeptide (II) but also the polypeptide having Met added to the N-terminus of (II) or a polypeptide equivalent to these in activities.

Such DNA can be prepared in the following manner. Human IFN-γ-secreting cells, such as human peripheral blood lymphocytes are cultivated and the human IFN-γ polypeptide-encoding mRNA is isolated from the culture by the known method.

Using the thus-obtained mRNA as the template, a DNA chain is synthesized by the per se known method using a reverse transcriptase, for instance, and the cDNA is converted to the double-stranded form [Maniatis, T. et al., Cell, 8, 163-182 (1976)].

This double-stranded DNA is inserted, for example, into the plasmid pBR322 at the PstI or SphI restriction endonuclease cleavage site, for example, by the dG-dC or dA-dT homopolymer tailing method [Nelson, T. S., Methods in Enzymology, 68, 41-50 (1979), Academic Press Inc., New York]. The recombinant plasmid is then introduced, for example, into Escherichia coli χ 1776, by transformation. The desired transformants can be selected as the tetracycline-resistant or ampicillin resistant colonies. Separately, an oligonucleotide having a base sequence supposedly corresponding to the amino acid sequence of the IFN-γ polypeptide is synthesized chemically and then labeled with $^{32}P$. Using the labeled oligonucleotide as the probe, the desired clones are secondarily screened out from among the already-obtained tetracycline- or ampicillin-resistant transformants by the per se known colony hybridization method [Grunstein, M. and Hogness, D. S., Proc. Natl. Acad. Sci. USA, 72, 3961-3965 (1975)], for instance.

For confirming the presence of the IFN-γ gene, the base sequence of the clones showing the positive colony hybridization result is determined, for example, by the

Maxam-Gilbert method [Maxam, A. M. and Gilbert, W., Proc. Natl. Acad. Sci. USA, 74, 560-564 (1977)] or the dideoxynucleotide synthetic chain termination method [Messing, J. et al., Nucleic Acids Research, 9, 309-321 (1981)]. Thereafter, the whole or part of the IFN-γ gene is excised from the clones obtained.

Furthermore, the DNA containing the base sequence of the formula

```
     1                                  10
    TGC TAC TGC CAG GAC CCA TAC GTG AAG GAA GCT GAA AAC
                            20
CTG AAG AAA TAC TTC AAC GCT GGT CAT TCT GAC GTT GCT GAC
        30                                      40
AAC GGT ACT CTG TTC CTG GGT ATC CTG AAA AAC TGG AAA GAA
                                50
GAA TCT GAC CGT AAA ATC ATG CAG TCT CAG ATC GTT TCT TTC
                60
TAC TTC AAG CTG TTC AAA AAC TTC AAG GAC GAC CAG TCT ATC
70                                          80
CAG AAA TCT GTT GAA ACT ATC AAG GAA GAC ATG AAC GTT AAG
                            90
TTC TTC AAC TCT AAC AAG AAA AAG CGT GAC GAC TTC GAA AAG
        100                                     110
CTT ACT AAC TAC TCT GTT ACT GAC CTT AAT GTA CAG CGT AAA
                                120
GCT ATC CAT GAA CTG ATC CAG GTT ATG GCT GAA CTG TCC CCG
                130
GCT GCT AAA ACT GGT AAG CGT AAA AGA TCT CAG ATG CTG TTC
140                     146
GGT GGT CGT CGT GCT TCT CAG
```

chemically synthesized by Tanaka, S. et al (vide supra) can also be used as the gene for the production of IFN-γ.

It is a known phenomenon that when, as a result of the expression of a certain foreign gene, the relevant protein is accumulated in a large amount, the growth of the host cells is inhibited and further the death of the cells may be caused. In order to avoid such phenomenon, it is desirable, if possible, to grow the cells in the condition in which the gene expression is suppressed and thereafter place the cells under adequate conditions for inducing the gene expression.

The *Escherichia coli* rec A promoter is a promoter which controls the synthesis of the rec A gene product.

The rec A gene plays a main role in the so-called SOS response [Witkin, E. M., Bacteriol. Reviews, 40, 869-907 (1976)] which involves the induced synthesis of its product and the repair of damaged DNA upon damage of DNA in cells or upon inhibition of replication. Its product (rec A protein) has protease activity. Since the lex A gene product acts as the rec A gene repressor, the expression of the rec A gene is normally suppressed. However, a DNA-damaging treatment, such as UV irradiation, nalidixic acid treatment or mitomycin treatment, strongly induces the synthesis of the rec A protein. This indicates that the rec A promoter is activated by such treatment.

Therefore, the recombinant DNA in accordance with the present invention with the human IFN-γ structural gene inserted downstream from the rec A promoter can be controlled as well, with respect to the synthesis of its product, by the above treatment. This property is very advantageous for the production of the human IFN-γ protein. The fact that the promoter activity is very strong further increases the utility of the present invention.

The base sequence of the rec A promoter has already been reported [Horii, T. et al., Proc. Natl. Acad. Sci. USA, 77, 313-317 (1980); Sancar, A. et al., ibid., 77, 2611-2615 (1980)]. However, there is no adequate restriction enzyme cleavage site downstream from the rec A promoter. Accordingly, it cannot be said to be advantageous to insert the structural gene downstream from the rec A promoter as it is. Since there is no restriction enzyme recognition site immediately following the rec A promoter, the ligation of the structural gene would unavoidably result only in the production of a chimera peptide of the rec A protein with the structure gene product. The present inventors newly prepared the rec A promoter, introduced various restriction enzyme recognition sites on both ends thereof, inserted the human IFN-γ structural gene downstream therefrom, and succeeded in reconstructing

the promoter such that the intended human IFN-γ structural gene can be expressed directly, not in the form of a chimera peptide between the rec A protein and the structural gene product.

Thus, the present invention also discloses, as a preferred embodiment thereof, a recombinant DNA which does not contain any other translational initiation codon than that for a human IFN-γ structural gene between the rec A promoter and said gene.

In the present specification, claims and drawings, the DNA represented by the nucleotide sequence:

CGGCGGGAATGCTTCAGCGGCGACCGTGATGCGGTGCGTCGTCAGGCTACTG
GCCGCCCTTACGAAGTCGCCGCTGGCACTACGCCACGCAGCAGTCCGATGAC

CGTATGCTTGCAGACCTTGTGGCAACAATTTCTACAAAACACTTGATACTGTATGA
GCATACGAACGTCTGGAACACCGTTGTTAAAGATGTTTTGTGAACTATGACATACT

GCATACAGTATAATTGCTTCAACAGAACATATTGACTATCCGGTATTACCCGGCAT
CGTATGTCATATTAACGAAGTTGTCTTGTATAACTGATAGGCCATAATGGGCCGTA

GACAGGAGTAAAA
CTGTCCTCATTTT

is referred to as "rec A promoter" for convenience' sake.

The symbols as used in the present specification and drawings each has the meaning given in Table 1.

Table 1

| | |
|---|---|
| DNA | deoxyribonucleic acid |
| A | deoxyadenylate |
| T | deoxythymidylate |
| G | deoxyguanylate |
| C | deoxycytidylate |
| RNA | ribonucleic acid |
| dATP | deoxyadenosine triphosphate |

| | |
|---|---|
| dTTP | deoxythymidine triphosphate |
| dGTP | deoxyguanosine triphosphate |
| dCTP | deoxycytidine triphsophate |
| ATP | adensoine triphosphate |
| EDTA | ethylenediaminetetraacetate |
| b | base(s) |
| bp | base pair(s) |
| Kbp | kilobase pairs |
| Kb | kilobases |
| $Apc^r$ | ampicilline resistance |
| $Tet^r$ | tetracycline resistance |
| recAp | rec A promoter |

The rec A promoter construction and the succeeding expression plasmid construction are performed, for example, in the following manner.

Double digestion of the plasmid pMCR611 DNA containing the rec A promoter inserted therein [Miki, T. et al., Mol. Gen. Genet., 183, 25-31 (1981)] with the restriction enzymes BamHI and PstI gives a 1.25 Kb DNA fragment containing the rec A promoter. Digestion of said fragment with the restriction enzyme HaeIII gives a 300 b DNA fragment containing the rec A promoter. Further digestion of this 300 b DNA fragment with the restriction enzyme HpaII gives a 148 b DNA fragment covering, according to the report of Sancar et al. (vide supra), the sequence from -129 to 19 with the RNA transcription startpoint on the rec A promoter being counted as 1. The SD sequence of the rec A promoter is missing in said DNA fragment.

Separately, oligonucleotides (dCGGTATTACCCGGC, dATGACAGGAGTAAAAG, dTCATGCCGGGTAATAC and dGATCCTTTTACTCCTG) are chemically synthesized, for example, by the phosphotriester method [Crea, R. et al., Proc. Natl. Acad. Sci. USA, 75, 5765-5769 (1978)]. These are ligated together, for example, using T4 DNA ligase. The thus-obtained 30 b DNA fragment is joined to the above 148 b DNA, for example

with T4 DNA ligase, to give a 178 b DNA fragment. The cohesive termini on both ends of this DNA are filled in, for example with DNA polymerase I large fragment and then the BamHI linker (dCCGGATCCGG) is joined to said DNA using T4 DNA ligase. The resulting DNA fragment is digested with the restriction enzyme BamHI and then inserted into the plasmid pBR322 digested with the same enzyme BamHI with the use of T4 DNA ligase. In this way, there can be constructed a recombinant plasmid, pREC3, which contains the rec A promoter possessing BamHI recognition sites on the ends thereof. An expression plasmid, pREC33, which possesses a BamHI site only downstream from the rec A promoter, can also be constructed by eliminating another BamHI site, which lies upstream from the rec A promoter, from said pREC3, for example by a series of operational steps, for example, partial digestion of pREC3 with BamHI, repair of the cohesive termini with DNA polymerase I large fragment and rejoining with T4 DNA ligase.

It is also possible to cut out the rec A promoter of pREC3 with the restriction enzyme BamHI, render the cohesive termini on both ends blunt with S1 nuclease, DNA polymerase I large fragment or the like and join thereto the EcoRI, ClaI or HindIII linker, for instance, whereby a DNA possessing a restriction enzyme recognition site suitable for the insertion of the structural gene into said site, which lies downstream from said promoter, can be added to the end of the promoter.

Furthermore, it is also possible, for example, to cut out, with a restriction enzyme, the rec A promoter of pREC3 or corresponding plasmids with the recognition sites at the ends of the promoter region being modified, ligate molecules of the cut-out promoter with each other with T4 DNA ligase and insert the thus-obtained successive dimer, trimer, etc. containing a plurality of rec A promoters into the plasmid pBR322, whereby an expression plasmid having still more potent promoter activity can be obtained.

In this case, it is preferable that there is no translational initiation codon between or among the rec A promoters.

Any IFN-γ structural gene mentioned above can be inserted, with the aid of a restriction enzyme and T4 DNA ligase, into the expression vector constructed in the above manner.

More concretely stating, a recombinant DNA containing a structural gene of human immune interferon downstream from a rec A promoter or promoters can be produced, for example, by combining a rec A promoter or promoters with a structural gene of human immune interferon downstream from said promoter or promoters in a plasmid.

Preferably, a recombinant DNA of the present invention can be produced by inserting a structural gene of human immune interferon downstream from a rec A promoter or promoters, by inserting a rec A promoter or promoters upstream from a structural gene of human immune interferon, or by inserting a rec A promoter or promoters upstream from a rec A promoter or promoters of the above obtained recombinant DNA in a plasmid.

The above expression plasmid or a recombinant DNA obtained by insertion of a structural gene for INF-γ thereinto can be introduced into an adequate host. The host includes microorganisms such as Escherichia coli, Bacillus subtilis and yeasts. Preferred are strains of Escherichia coli (strains 294, W3110, RR1, DM511, DM1187, etc.), in particular strain 294. The DM511 strain containing mutation in the lex A gene which is the repressor against the rec A promoter [Mount, D. W. et al., J. Bacteriol., 116, 950-956 (1973)] and the DM1187 strain [Mount, D. W., Proc. Natl. Acad. Sci. USA, 74, 300-304 (1977)] are also suitable for the development of activity by the present promoter.

The strain 294 is a known strain [Backman, K. et al., Proc. Natl. Acad. Sci. USA, 73, 4174-4178 (1976)]. It is

deposited at Institute for Fermentation, Osaka under No. IFO-14171.

The transformation of a host with the above recombinant DNA can be carried out by the known method [Cohen, S. N. et al., Proc. Natl. Acad. Sci. USA, 69, 2110-2114 (1972)] or a modification thereof.

The thus-obtained transformant is cultivated in a known medium, such as M9 medium containing glucose and casamino acids [Miller, J., Experiments in Molecular Genetics, 431-433 (Cold Spring Harbor Laboratory, New York), 1972]. For efficient functioning of the promoter, in other words for suppressing the inhibition of the rec A gene expression, such an agent as mitomycin C or nalidixic acid may be added or ultraviolet irradiation may be made as necessary in carrying out the cultivation. The cultivation is conducted generally at 15° to 43°C for 3 to 24 hours. Aeration and/or stirring may be made as necessary. After cultivation, cells are harvested by the known method, then suspended for example in a buffer solution, and destructed for example by treatment with ultrasonic waves or an enzyme such as lysozyme. The supernatant is collected by the known method, such as centrifugation.

The content of IFN-γ in said supernatant can be determined for antiviral activity by the cytopathic effect inhibition using vesicular stomatitis virus (VSV) on human amnion-derived WISH cells [Stewart, W. E., The Interferon System, 11-26 (Springer Verlag, New York), 1979].

For isolating human IFN-γ from such IFN-γ-containing supernatant, there may be used any of generally known methods of purifying proteins. In particular, the purification method using an antibody capable of binding to human IFN-γ is advantageous. Thus, for example, the purification can advantageously be effected by using a monoclonal antibody against the peptide H-Lys-Arg-Lys-Arg-Ser-Gln-Met-Leu-Phe-Arg-Gly-Arg-Arg-Ala-Ser-Gln-OH. Said monoclonal antibody can advantageously be used in

the form of an antibody column.

The antibody column can be prepared by coupling the above-mentioned monoclonal antibody purely isolated from hybridoma-inoculated ascites, for instance, to an adequate carrier, in the manner such as mentioned below.

The carrier to be used may be any of those which, after coupling, can adsorb IFN-γ selectively and efficiently and thereafter allows adequate elution of IFN-γ. For example, agarose gel beads activated such that the primary amino group of proteins can easily be bound thereto, such as AFFI-GEL 10 (Bio-Rad Lab., USA) can be favorably used in the following manner. The reaction of AFFI-GEL 10 with the antibody is carried out in a buffer solution, such as 0.001-1 M, preferably 0.1 M, bicarbonate. Usable reaction conditions are 0°-20°C, 10 minutes to 24 hours, and varying pH, and preferred conditions are 4°C, 4 hours and pH 3-10. The quantitative ratio between AFFI-GEL 10 and the antibody may be selected within the range of up to about 50 mg of antibody per ml of AFFI-GEL, since, in this range, the amount of antibody coupled with AFFI-GEL increases as the amount of antibody increases. From the view points of the coupling efficiency and the purification efficiency in the affinity chromatography, the antibody is preferably used in an amount between 1 mg and 30 mg per ml of AFFI-GEL. The thus-produced antibody-carrier coupling product is washed well with the same buffer solution as used for the reaction and then allowed to stand for several days or treated with ethanolamine hydrochloride in a final concentration of 0.05 M at 4°C for an hour or by some other method so as to block the remaining unreacted active groups, and packed in an appropriate column as is per se known in the art to give an antibody column.

In using the above antibody column for purification, a human immune interferon protein-containing solution is dissolved in an almost neutral buffer, such as phosphate buffer or tris hydrochloride buffer, and subjected to

adsorption of the antibody column. The column is then washed with the same buffer, followed by elution of IFN-γ. The eluent is, for example, a weakly acidic solution (e.g. acetic acid solution), a polyethylene glycol-containing solution, a solution containing a peptide capable of being more easily bound to the antibody as compared with the sample, a high-concentration salt solution, or a mixed solution composed of these, which should preferably be incapable, as far as possible, of causing decomposition of human IFN-γ.

The eluate from the column is neutralized with a buffer by the conventional method. As necessary, it may be subjected again to the above antibody column purification.

The thus-obtained human IFN-γ protein solution is dialyzed. The dialysate may be made into a powder by lyophilization, as necessary. In performing such lyophilization, a stabilizing agent, such as sorbitol, mannitol, dextrose, maltose or glycerol, may be added.

The thus-obtained human immune interferon protein, when determined for antiviral activity by the cytopathic inhibition assay using the vesicular stomatitis virus (VSV) on human amnion-derived WISH cells, shows a specific activity of not less than $10^7$ U/mg.

The human immune interferon protein produced in accordance with the present invention can be used for the same purposes and in the same manner of use as in the case of IFN-γ species obtained by the conventional methods. As compared with the conventional products, it contains smaller amounts of contaminant proteins and pyrogens and accordingly can be used more safely as a bulk substance for injectable preparations.

The human IFN-γ protein according to the present invention has antiviral, antitumor, antiproliferative and immunopotentiating activities. The human IFN-γ protein in accordance with the invention can be mixed with a known,

physiologically acceptable carrier, such as sterilized water, human serum albumin (HSA) or physiological saline, for parenteral or local administration. Thus, for instance, it can be administered intravenously or intramuscularly.

The preparation containing the human IFN-γ protein according to the invention may also contain other physiologically acceptable active ingredients such as salt, diluent, adjuvant, other carrier, buffer, binding agent, surfactant and preservative. The preparation for parenteral administration is supplied in the form of a sterile powder (generally obtainable by lyophilization of an IFN-γ solution) in ampuls, which can be used after dilution with a physiologically acceptable diluent.

Furthermore, the preparation containing the human IFN-γ protein according to the invention may contain another active ingredient, such as IFN-α or IFN-β or a lymphokine such as interleukin 2, in an amount of 1-99 percent based on the substance according to the present invention.

## Brief Description of the Drawings

Fig. 1 shows the restriction enzyme cleavage map of the plasmid pHIT3709 as obtained in Reference Example 1-(vii), the portion 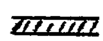 indicating the portion coding for a peptide supposed to be the signal peptide and the portion 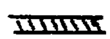 indicating the portion coding for the IFN-γ polypeptide. Fig. 2 shows the primary structure (base sequence) of the plasmid pHIT3709 as obtained in Reference Example 1-(vii). The base sequence 1-146 illustrates a mode of the human immune interferon structural gene.

Fig. 3 illustrates the construction scheme for the plasmid pREC3 as described in Reference Example 2, Fig. 4 for the rec A promoter-containing expression vector (Reference Example 3), Fig. 5 for the plasmid pHITrec1202 (Example 1) and Fig. 6 for the plasmids pHITrec1208, pHITrec2202 and pHITrec3202 (Example 2).

The present invention will be further explained by way of the following reference and working examples, but these examples should be understood not to limit thereby the present invention.

Escherichia coli 294/pREC206 disclosed in Reference Example 3 is deposited at Institute for Fermentation, Osaka (IFO) under the deposit No. 14320.

Reference Example 1

(i) Isolation of mRNA coding for human IFN-γ

Lymphocytes prepared from the human peripheral blood were incubated at 37°C in the RPMI-1640 medium (containing 10% fetal calf serum) containing 15 ng/ml of 12-O-tetradecanoylphorbol-13-acetate (TPA) and 40 μg/ml of concanavalin A for IFN-γ induction. After twenty-four (24) hours, the thus-induced human lymphocytes (1 x $10^{10}$ cells) were destructed in a thioguanidine solution (5M guanidine thiocyanate, 5% mercaptoethanol, 50 mM Tris HCl, pH 7.6, 10 mM EDTA) in a Teflon homogenizer. Then, sodium N-lauroyl sarcosinate was added in the concentration of 4% and the mixture after homogenization was layered over 6 ml of 5.7 M cesium chloride [5.7 M cesium chloride, 0.1 M ethylenediaminetetraacetate (EDTA)] and centrifuged at 15°C and 24000 rpm for 30 hours using a Beckman SW27 rotor to give an RNA precipitate. This RNA precipitate was dissolved in 0.25% sodium N-lauroyl sarcosinate and then precipitated with ethanol to give 8.3 mg of RNA. This RNA was allowed to be absorbed, in a high-concentration salt solution (0.5M-NaCl, 10 mM-Tris HCl; pH 7.6, 1 mM-EDTA, 0.3% SDS), on oligo (dT) cellulose column and mRNA containing poly (A) was eluted with a low-concentration salt solution (10 mM-Tris HCl, pH 7.6, 1 mM EDTA, 0.3% SDS) to give 700 μg of mRNA. This mRNA was further precipitated with ethanol, then dissolved in 0.2 ml of a solution (10 mM Tris HCl, pH 7.6, 2 mM EDTA, 0.3% SDS), treated at 65°C for 2 minutes, and fractionated by 10-35% sucrose density

gradient centrifugation at 20°C and 25000 rpm for 21 hours using a Beckman SW27 rotor, to give 22 fractions. Aliquots of each fraction were injected into Xenopus laevis oocytes and the proteins synthesized were assayed for interferon activity [antiviral activity as determined by the cytopathic effect inhibition assay using vesicular stomatitis virus on WISH cells derived from human amnion [Stewart, W.E. vide supra]. In this manner, it was found that fraction 12 (the sedimentation constant being 12-14S) had an activity of 195 units per μg of RNA. The mRNA in the thus-obtained fraction 12 weighed about 20 μg.

(ii) Synthesis of single-stranded DNA

Using the above mRNA and a reverse transcriptase, 100 μl of a reaction mixture (5 μg of mRNA, 50 μg of oligo (dT), 100 units of reverse transcriptase, 1 mM each of dATP, dCTP, dGTP and dTTP, 8 mM $MgCl_2$, 50 mM KCl, 10 mM dithiothreitol and 50 mM Tris HCl; pH 8.3) was incubated at 42°C for 1 hour, then, deproteinized by adding phenol and treated with 0.1N NaOH at 70°C for 20 minutes for removal of RNA by decomposition.

(iii) Synthesis of double-stranded DNA

The thus-synthesized single stranded complementary DNA was subjected to reaction in 50 μl of a reaction mixture (the same mixture as above except that the mRNA and oligo dT were absent) at 42°C for 2 hours for synthesizing the double-stranded DNA.

(iv) Addition of dC tails

The double-stranded DNA was treated with nuclease S1 in 50 μl of a reaction mixture (double-stranded DNA, 0.1 M sodium acetate, pH 4.5, 0.25 M NaCl, 1.5 mM $ZnSO_4$, 60 units S1 nuclease) at room temperature for 30 minutes. The reaction mixture was deproteinized by adding phenol and DNA was precipitated with ethanol. The thus-obtained DNA was reacted with terminal transferase in 50 μl of a reaction mixture (double-stranded DNA, 0.14 M potassium cacodylate, 0.3 M Tris (base) (pH 7.6), 2 mM dithiothreitol,

1 mM $CoCl_2$, 0.15 mM dCTP, 30 units terminal transferase) at 37°C for 3 minutes for elongation of the double-stranded DNA by about 20 deoxycytidine (about 20) units at each 3'-end of the DNA. This series of reactions gave about 300 ng of deoxycytidine-tailed double-stranded DNA.

(v) Cleavage of *Escherichia coli* plasmid and addition of dG tails

Separately, 10 μg of *Escherichia coli* plasmid pBR 322 DNA was treated with restriction enzyme *Pst*I in 50 μl of a reaction mixture [10 μg DNA, 50 mM NaCl, 6 mM Tris HCl (pH 7.4), 6 mM $MgCl_2$, 6 mM 2-mercaptoethanol, 100 μg/ml bovine serum albumin, 20 units *Pst*I] at 37°C for 3 hours for cleavage at the one *Pst*I recognition site present in the pBR322DNA, the reaction mixture was then deproteinized with phenol and the DNA was further subjected to terminal transferase treatment in 50 μl of a reaction mixture [10 μg DNA, 0.14 M potassium cacodylate, 0.3 M Tris (base) pH 7.6, 2 mM dithiothreitol, 1 mM $CoCl_2$, 0.15 mM dGTP, 30 units terminal transferase at 37°C for 3 minutes for elongation of the above pBR322 plasmid DNA by about 8 deoxyguanidines at each 3'-end thereof.

(vi) Annealing of cDNA and transformation of *Escherichia coli*

The annealing was effected by heating 0.1 μg of the thus-obtained synthetic double-stranded DNA and 0.5 μg of the above pBR322 plasmid in a solution containing 0.1M NaCl, 50 mM Tris HCl pH 7.6, 1 mM EDTA at 65°C for 2 minutes and then at 45°C for 2 hours, followed by gradual cooling. The transformation of *Escherichia coli* χ1776 was performed by the method of Enea et al. [J. Mol. Biol., 96, 495-509 (1975)].

(vii) Isolation of cDNA-containing plasmid

About 8,500 tetracycline-resistant colonies were thus isolated and the DNA of each colony was fixed to a nitrocellulose filter [M. Grunstein and D.S. Hogness, vide supra].

Separately, based on the amino acid sequence of IFN-γ as reported by D.V. Goeddel et al. [Nature, <u>295</u>, 503-509 (1982)], two oligonuclotides of
A A
5'TCCTGGCAGTAGC3' and
G G T T
5'ACATTCATATCCTCCT3' presumably corresponding to amino acids Nos. 1-5 (Cys. Tyr. Cys. Gln. Asp) and amino acids Nos. 77-82 (Lys. Gln. Asp. Met. Asn. Val) of said IFN-γ sequence, respectively, were chemically synthesized by the triester method [R. Crea et al., vide supra]. These oligonucleotides were treated with T4 polynucleotide kinase in 50 μl of a reaction mixture (0.2 μg oligonucleotide, 50 mM Tris HCl pH 8.0, 10 mM $MgCl_2$ 10 mM mercaptoethanol, 50μCiγ-$^{32}$P ATP, 3 units T4 polynucleotide kinase) at 37°C for an hour. These oligopeptides thus labeled with $^{32}$P at the 5'-end were used as probes and annealed with the DNA on the above-mentioned nitrocellulose filter by the method of Lawn et al. [Nucleic Acids Res., <u>9</u>, 6103-6114 (1981)]. Four colonies were isolated by autoradiography which were reactive to the above two oligonucleotide probes.

Plasmid DNAs were isolated from the bacterial cells of each of these strains by the alkali method [H.C. Birnboim and J. Doly, Nucleic Acids Res., <u>7</u>, 1513-1523 (1979)]. The inserts in the plasmid DNAs were excised with the <u>PstI</u> restriction enzyme. From among the isolated plasmids, the one containing the longest insert was chosen and named "pHIT3709".

The structure (base sequence) of the cDNA sequence inserted in the pHIT3709 plasmid was then determined by the dideoxynucleotide synthetic chain termination method and by the Maxam-Gilbert method. Said primary structure was as shown in Fig. 2.

## <u>Reference Example 2</u>

Preparation of rec A promoter-containing DNA fragment (cf. Fig. 3)

The plasmid pMCR611 (300 μg) (Miki, T et al. vide

supra) was digested with the restriction enzymes BamHI and PstI (Takara Shuzo). Agarose gel (1%) electrophoresis gave a 1.25 kbp DNA fragment containing the rec A promoter portion, which fragment was eluted from the gel [McDonell, M. W. et al., J. Mol. Biol., 110, 119-146 (1977)]. This DNA fragment was purified by phenol extraction, ether extraction and ethanol precipitation, then digested with the restriction enzyme HaeIII (Takara Shuzo), and fractionated by 1.5% agarose gel electrophoresis to give a 300 b DNA fragment containing the rec A promoter portion. The fragment was purified in the same manner as mentioned above. This DNA fragment was further digested with the restriction enzyme HpaII (Takara Shuzo). The digestion product was subjected to 8% acrylamide gel (acrylamide: bisacrylamide= 19:1) electrophoresis so as to isolate the 148 b DNA fragment covering the sequence from -129 to 19 with the RNA transcription startpoint on the rec A promoter being counted as 1 as reported by Sancar et al. (vide supra). The thus-fractionated fragment was purified in the same manner as above.

Separately, four oligonucleotides each containing a portion of the 3'-end of the rec A promoter, namely (1) dCGGTATTACCCGGC, (2) dATGACAGGAGTAAAAG, (3) dTCATGCCGGGTAATAC and (4) dGATCCTTTTACTCCTG, were chemically synthesized by the phosphotriester method (vide supra). Of these oligonucleotides, (2) and (3) were phosphorylated at the 5'-end using polynucleotide kinase [New England Biolabs (NEB)] and ATP. Equal amounts (0.5 μg) of the four oligonucleotides, namely oligonucleotides (1) and (4) and 5'-phosphorylated oligonucleotides (2) and (3), were mixed and, using T4 DNA ligase, they were ligated to one another by maintaining the mixture in 50 μl of reaction medium (66 mM Tris-HCl, pH 7.6, 6.6 mM $MgCl_2$, 10 mM dithiothreitol, 1 mM ATP, 300 units T4 DNA ligase) at 14°C for 16 hours. The reaction product was fractionated by 10% acrylamide gel (acrylamide : bisacrylamide=19:1)

electrophoresis. The 30 b DNA fragment, a ligation product of the oligonucleotides, was isolated and purified in the same manner as mentioned above.

In the next place, the above-mentioned 148 b DNA fragment and the 30 b DNA fragment were ligated together using T4 DNA ligase. The reaction product was fractionated by 8% acrylamide gel electrophoresis. The 178 b DNA fragment which thus formed was isolated and purified. The cohesive termini of this DNA fragment were rendered blunt by contacting the DNA fragment with DNA polymerase I large fragment [Bethesda Research Laboratories (BRL)] in 30 µl of reaction mixture (40 mM potassium phosphate buffer, pH 7.5, 6.6 mM $MgCl_2$, 1 mM 2-mercaptoethanol, 20 µM dATP, 20 µM dGTP, 20 µM dCTP, 20 µM dTTP, 2 units DNA polymerase I large fragment) at 20°C for 30 minutes. The product DNA was purified by phenol extraction and ethanol precipitation and thereto was joined the BamHI linker dCCGGATCCGG (BRL) phosphorylated at the 5'-end, using T4 DNA ligase. The product was digested with the restriction enzyme BamHI and inserted into the pBR322 DNA BamHI digestion product. The resultant recombinant DNA was used for transformation of Escherichia coli strain 294. Ampicillin-resistant, tetracycline-sensitive transformants were selected and plasmid DNAs were isolated therefrom according to the method of Birnboim-Doly [vide supra]. A plasmid which gave a 185 b DNA fragment upon digestion with the restriction enzyme BamHI was selected. At the same time, the base sequence of the 185 b DNA fragment thus cut out was determined according to the dideoxynucleotide synthetic chain termination method [vide supra]. In this manner, there was obtained the product of ligation of the 148 b DNA fragment from the 5'-end portion of the rec A promoter with the BamHI linker as joined together in the expected manner. Its nucleotide sequence was as follows:

```
     -130      -120      -110      -100       -90       -80
GATCCGGCGGCGGGAATGCTTCAGCGGCGACCGTGATGCGGTGCGTCGTCAGGCTACT
    GCCGCCGCCCTTACGAAGTCGCCGCTGGCACTACGCCACGCAGCAGTCCGATGA

       -70       -60       -50       -40       -30
GCGTATGCTTGCAGACCTTGTGGCAACAATTTCTACAAAACACTTGATACTGTATGA
CGCATACGAACGTCTGGAACACCGTTGTTAAAGATGTTTTGTGAACTATGACATACT

-20       -10         1        10        20       30
GCATACAGTATAATTGCTTCAACAGAACATATTGACTATCCGGTATTACCCGGCATG
CGTATGTCATATTAACGAAGTTGTCTTGTATAACTGATAGGCCATAATGGGCCGTAC

   40
ACAGGAGTAAAAG
TGTCCTCATTTTCCTAG
```

As compared with the report of Sancar, A et al. [vide supra], the above sequence contains one more base pair $(\frac{A}{T})$ at -121.

A pBR322 plasmid with this DNA fragment inserted therein with the same orientation of the rec A promoter with that of the ampicillin resistance-offering β-lactamase gene was named pREC3.

## Reference Example 3

Construction of rec A promoter-containing expression vector (cf. Fig. 4)

The plasmid pREC3 was digested with the restriction enzyme BamHI, followed by 6% acrylamide gel electrophoresis, which gave a 185 b rec A promoter unit DNA. The cohesive termini of this DNA were rendered blunt with S1 nuclease (BRL) in 100 μl of reaction mixture (0.5 μg DNA, 0.03 M sodium acetate buffer, pH 4.5, 0.3 M NaCl, 4.5 mM $ZnCl_2$, 3 units S1 nuclease) at 22°C for 30 minutes and then the DNA was purified by phenol extraction and ethanol precipitation. The EcoRI linker dGGAATTCC (BRL)

was phosphorylated at the 5'-end and ligated with the above DNA using T4 DNA ligase. The ligation product was digested with the restriction enzyme EcoRI (Takara Shuzo) and ligated with the pBR322 DNA digested with the same enzyme. The ligation product was used for transformation of Escherichia coli strain 294. Ampicillin-resistant and tetracycline-resistant transformants were selected. Plasmid DNAs were isolated from these transformants according to the method of Birnboim-Doly (vide supra). Based on the restriction enzyme cleavage pattern, a plasmid (pREC206) containing the rec A promoter in the same orientation as that of the β-lactamase gene and a plasmid (pREC207) containing the rec A promoter in the opposite orientation to the β-lactamase gene were selected.

Escherichia coli 294 was transformed with the plasmid pREC206 to obtain Escherichia coli 294/pREC206.

## Example 1

The plasmid pHIT3709 obtained in Reference Example 1 was cleaved with the restriction enzyme PstI to give a PstI fragment containing the structural gene for IFN-γ. This fragment was partially cleaved with the restriction enzyme BstNI (NEB) to give a BstNI-PstI fragment cleaved at the BstNI site within the IFN-γ structural gene. The BstNI cleavage site was filled in with DNA polymerase I large fragment, followed by ligation with the oligonucleotide adapter

AATTCATGTGTTATTGTC
GTACACAATAACAG

chemically synthesized by the phosphotriester method mentioned above and containing the protein synthesis start codon ATG, using T4 DNA ligase.

Separately, the plasmid pREC206 obtained in Reference Example 3 was digested with the restriction enzyme PstI and further subjected to partial digestion with the restriction enzyme EcoRI. The digestion product was

subjected to 1% agarose gel electrophoresis and a 3.8 Kb DNA fragment was isolated and purified by the method described in Reference Example 2.

This DNA and the above-mentioned IFN-γ structural gene-oligonucleotide adapter ligation product were ligated together using T4 DNA ligase.

The thus-produced ligation product was used for transformation of Escherichia coli strain 294, and tetracycline-resistant transformants were selected. Plasmid DNAs were isolated from these transformants by the Birnboim-Doly method (vide supra) and, based on the restriction enzyme cleavage pattern, an expression plasmid pHITrec1202 with the rec A promoter, oligonucleotide adapter and human IFN-γ structural gene inserted in succession therein was selected. (Cf. Fig. 5).

Example 2 (Cf. Fig. 6)

(i) The plasmid pHITrec1202 obtained in Example 1 was partially cleaved with the restriction enzyme EcoRI so as to convert the circular DNA to the linear form. The resultant EcoRI cohesive termini were repaired with DNA polymerase I large fragment. This DNA was reconverted to the circular form using T4 DNA ligase and used for transformation of Escherichia coli strain 294. Tetracycline-resistant transformants were selected and plasmid DNAs were isolated therefrom by the Birnboim-Doly method (vide supra). Based on the restriction enzyme cleavage pattern, an IFN-γ expression plasmid, pHITrec1208, which did not contain the EcoRI recognition site originally found in pHITrec1202 at the junction site between the rec A promoter and IFN-γ gene any more, was selected.

(ii) pHITrec1208 was cleaved with the restriction enzymes EcoRI and PstI, the cleavage product was subjected to 1.2% agarose gel electrophoresis, and a 1.2 Kb DNA fragment was isolated and purified by the procedure described in Reference Example 1.

Separately, pREC206 was digested with the restriction enzyme PstI and further subjected to partial digestion with the restriction enzyme EcoRI, followed by 1% agarose gel electrophoresis of the digestion product, and a 3.8 Kb DNA fragment containing the rec A promoter portion was isolated and purified by the procedure described in Reference Example 1.

This DNA and the above-mentioned, pHITrec1208-derived 1.2 Kb DNA containing a rec A promoter and IFN-γ gene were ligated together using T4 DNA ligase and the ligation product was used for transformation of Escherichia coli strain 294. Tetracycline-resistant transformants were selected and plasmid DNAs were isolated therefrom by the Birnboim-Doly method (vide supra). Based on the restriction enzyme cleavage pattern, an IFN-γ expression plasmid, pHITrec2202, with two rec A promoter-containing DNA fragments inserted upstream from the IFN-γ gene in the same orientation as said gene was selected.

(iii) pHITrec2202 was subjected to digestion with the restriction enzyme PstI and further to partial cleavage with the restriction enzyme EcoRI, followed by 1.2% agarose gel electrophoresis, and a 1.4 Kb DNA fragment containing two rec A promoters and the IFN-γ gene was isolated and purified by the procedure described in Reference Example 1.

This DNA and the above-mentioned, pREC-206-derived, a rec A promoter containing 3.8 Kb EcoRI-PstI DNA fragment were ligated together and the ligation product was used for transformation of Escherichia coli strain 294. Tetracycline-resistant transformants were selected and plasmid DNAs were isolated therefrom by the Birnboim-Doly method (vide supra). Based on the restriction enzyme cleavage pattern, an expression plasmid, pHITrec3202, with three rec A promoters connected in series as a trimer and inserted therein upstream from the IFN-γ gene in the same directionality as that of said gene was selected.

Example 3

Escherichia coli 294 transformants carrying the IFN-γ expression plasmids constructed in Examples 1 and 2, namely pHITrec1202, 1208, 2202 and 3202, respectively were grown in 200-ml Erlenmeyer flasks each containing 10 ml of M9 medium (vide supra) containing 8 μg/ml of tetracycline, 0.4% of casamino acids and 1% of glucose at 37°C. When the growth reached KU 200, nalidixic acid was added to a concentration of 50 μg/ml and the cultivation was continued for further 5 hours. Cells were harvested from each culture by centrifugation and suspended in 1 ml of 0.05 M Tris-HCl pH 7.6 containing 10% of sucrose. To this suspension, there were added 5 μl of 0.2 M phenylmethylsulfonyl fluoride (PMSF), 40 μl of 5 M NaCl, 40 μl of 0.2 M EDTA, 40 μl of 1 M spermidine hydrochloride and 20 μl of 10 mg/ml lysozyme. After standing at 0°C for 1 hour, the mixture was treated at 37°C for 3 minutes. This lysate was centrifuged at 15,000 rpm (Servall centrifuge SS 34 rotor) for 30 minutes. The supernatant thus obtained was assyed for the antiviral activity by the test for inhibition of the cytopathic effect of vesicular stomatitis virus (VSV) on human amnion-derived WISH cells. The results obtained are given below.

| | Antiviral activity |
|---|---|
| 294/pHITrec1202 | $1.8 \times 10^8$ U/liter broth |
| 1208 | $3.5 \times 10^8$ |
| 2202 | $1.8 \times 10^8$ |
| 3202 | $3.5 \times 10^8$ |

Example 4

The IFN-γ expression plasmids pHITrec 1202, 1208, 2202 and 3202 were used for transformation of Escherichia coli DM1187 and tetracycline-resistant transformants were selected.

The IFN-γ-expression plasmid-containing Escherichia coli DM1187 transformants were cultured in 200-ml Erlenmeyer flasks each containing 10 ml of M9 medium containing 8 μg/ml of tetracycline, 0.4% of casamino acids and 1% of glucose at 37°C for 10 hours. Cells were collected from the culture broth by centrifugation. The supernatant prepared by the procedure described in Example 3 was assayed for the antiviral activity. The results obtained are given below.

| | Antiviral activity |
|---|---|
| DM1187/pHITrec1202 | $3.5 \times 10^8$ U/liter broth |
| 1208 | $1.8 \times 10^8$ |
| 2202 | $3.5 \times 10^8$ |
| 3202 | $1.8 \times 10^8$ |

Example 5

Escherichia coli DM1187/pHITrec2202 was cultured in one-liter Erlenmeyer flasks each containing 200 ml of M9 medium containing 8 μg/ml of tetracycline, 0.4% of casamino acids and 1% of glucose at 37°C for 10 hours.

The culture broth obtained (1.2 liters) was centrifuged and the cells thus collected were suspended in 60 ml of 0.05 M Tris-HCl pH 7.6 containing 10% of sucrose. To this cell suspension, there were added 0.3 ml of 0.2 M phenylmethylsulfonyl fluoride (PMSF), 2.4 ml of 5 M NaCl, 2.4 ml of 0.2 M ethylenediaminetetraacetate (EDTA), 2.4 ml of 1 M spermidine and 2.4 ml of 5 mg/ml lysozyme. After standing at 0°C for 1 hour, the mixture was treated at 37°C for 5 minutes and further treated on a sonicator (ARTEK, USA) at 0°C for 30 seconds.

This lysate was centrifuged at 105,000 x g for 1 hour. The supernatant thus collected amounted to 66 ml.

This supernatant (60 ml) was diluted with 20 mM Tris-HCl, pH 7.6 (TEN) containing 1 mM EDTA and 0.15 M

NaCl to make 150 ml and submitted to an anti-IFN-γ antibody column (8 ml) prepared by the procedure described in Reference Example 10 contained in the specification of patent application 83 109 403.2 claiming the priority of PCT/JP82/00445 by the present applicant. After adequate washing with TEN, the column was further washed with TEN containing 0.01% Nonidet P-40 (Shell) and 0.5 M NaCl. Thereafter, IFN-γ was eluted with 0.1 M acetic acid containing 0.25 M NaCl and the eluate was immediately neutralized with 1 M Tris-HCl pH 7.6. The solution thus obtained was dialyzed against distilled water at 4°C for 16 hours and the dialysate was frozen with acetone-dry ice and lyophilized to give a powder.

The results obtained are summarized below.

| | Protein (mg) | Total activity (U) | Specific activity (U/mg) | Recovery (%) |
|---|---|---|---|---|
| Lysate supernatant | 225.8 | $2.4 \times 10^8$ | $1.1 \times 10^6$ | - |
| Antibody column treatment | 1.8 | $8 \times 10^7$ | $4.4 \times 10^7$ | 33.3 |

The human immune interferon protein finally obtained in this example had a specific activity of $4.4 \times 10^7$ U/mg (by the test for inhibition of the cytopathic effect of VSV on WISH cells; vide supra). This protein was submitted to the experiments mentioned below.

## Example 6

Properties of human immune interferon protein

(i) Molecular weight

The protein obtained in Example 5 was treated with 2-mercaptoethanol and subjected to SDS-polyacrylamide gel (17.5%) electrophoresis (15 mV, 6 hours). Upon coomassie blue staining, the protein could be confirmed as a single band. Based on the relation between the distance of migration of a molecular weight marker electrophoresed

simultaneously and the distance of migration of the protein, the molecular weight of the protein was estimated at 17,000 ± 1,000. On the other hand, when the protein was not treated with 2-mercaptoethanol, another band was detected at a position corresponding to the molecular weight of 33,000 ± 2,000. Since this value is about two times the molecular weight of IFN-γ, i.e. 17,000 ± 1,000, this band is supposedly due to the dimer of INF-γ.

(ii) Amino acid analysis

A portion of the protein obtained in Example 5 was placed in a glass test tube for hydrolysis. After addition of constant boiling point hydrochloric acid containing 200 volumes (v/w) of 4% thioglycolic acid, the tube was sealed under reduced pressure. The hydrolysis was conducted at 110°C for 24, 48 or 72 hours. Thereafter, the tube was opened, the hydrochloric acid was removed under reduced pressure, and the residue was dissolved in 0.02 N hydrochloric acid and subjected to amino acid analysis on a Hitachi model 835 high-speed amino acid analyzer.

As for cystine and cysteine, they were determined together by the method of Hirs [Methods in Enzymology, 11, 197-199 (1967)], namely in the form of cysteic acid on the amino acid analyzer following performic acid oxidation of the protein and the same 24-hour hydrolysis as above. The amino acid analysis values obtained after 24, 48 and 72 hours of hydrolysis were averaged except for serine, threonine, tyrosine and tryptophan, the values for which were determined by extrapolation of the hydrolysis time to 0 hour. The results obtained are shown in Table 2.

Table 2

| Amino acid detected | Mol % | Amino acid detected | Mol % |
|---|---|---|---|
| Aspartic acid | 13.5 | Methionine | 2.8 |
| Threonine | 3.6 | Isoleucine | 4.8 |
| Serine | 7.6 | Leucine | 6.8 |
| Glutamic acid | 12.3 | Tyrosine | 3.3 |
| Proline | 1.2 | Phenylalanine | 6.9 |
| Glycine | 3.1 | Lysine | 13.8 |
| Alanine | 5.3 | Histidine | 1.3 |
| Cysteic acid | 1.4 | Arginine | 5.7 |
| Valine | 5.6 | Tryptophan | 0.8 |

(iii) Amino-terminal amino acid analysis

The protein obtained in Example 5 was oxidized with performic acid by the method of Hirs (vide supra), and the amino-terminal amino acid analysis thereof was performed by the Edman degradation method modified by Iwanaga et al. [Eur. J. Biochem., 8, 189-199 (1969)]. The phenylthiohydantoinamino acids (PTH-amino acids) were identified and assayed by the method of Archer [Altex Chromatogram, 3, 8 (1980)] on a Varian (USA) model 5040 high-performance liquid chromatograph using an Ultrasphere ODS column [Altex (USA), 4.6 x 250 mm, grain size 5 μm]. As a result, PTH-methionine sulfone and PTH-cysteic acid were detected.

What is claimed is:

1. A recombinant DNA which contains a structural gene of human immune interferon downstream from a rec A promoter or promoters.

2. A DNA according to Claim 1 which does not have any other translational initiation codon than that for a structural gene of human immune interferon between the rec A promoter and the structural gene.

3. A DNA according to Claim 1 or 2, which has one rec A promoter.

4. A DNA according to Claim 1 or 2, which has a plurality of rec A promoters.

5. A DNA according to Claim 4, which does not have any translational initiation codon between the rec A promoters.

6. A DNA according to Claim 1, wherein the structural gene of human immune interferon is of the base sequence of the formula:

(5') TGT TAC TGC CAG GAC CCA TAT GTA AAA GAA GCA GAA AAC
CTT AAG AAA TAT TTT AAT GCA GGT CAT TCA GAT GTA GCG GAT
AAT GGA ACT CTT TTC TTA GGC ATT TTG AAG AAT TGG AAA GAG
GAG AGT GAC AGA AAA ATA ATG CAG AGC CAA ATT GTC TCC TTT
TAC TTC AAA CTT TTT AAA AAC TTT AAA GAT GAC CAG AGC ATC
CAA AAG AGT GTG GAG ACC ATC AAG GAA GAC ATG AAT GTC AAG
TTT TTC AAT AGC AAC AAA AAG AAA CGA GAT GAC TTC GAA AAG
CTG ACT AAT TAT TCG GTA ACT GAC TTG AAT GTC CAA CGC AAA
GCA ATA CAT GAA CTC ATC CAA GTG ATG GCT GAA CTG TCG CCA
GCA GCT AAA ACA GGG AAG CGA AAA AGG AGT CAG ATG CTG TTT
CGA GGT GGA AGA GCA TCC CAG (3')

7. A method of producing a recombinant DNA containing a structural gene of human immune interferon downstream from a rec A promoter or promoters, which comprises combining a rec A promoter or promoters with a structural gene of human immune interferon drownstream from said promoter or promoters in a plasmid.

8. A transformant which contains a recombinant DNA having a structural gene of human immune interferon downstream from a rec A promoter or promoters.

9. A transformant according to Claim 8, which is *Escherichia coli*.

10. A transformant according to Claim 9, which is *Escherichia coli*, of which repressing function against rec A gene expression is lessened.

11. A method of producing a transformant containing a recombinant DNA having a structural gene of human immune interferon downstream from a rec A promoter or promoters, which comprises transforming a host organism with said recombinant DNA.

12. A method according to Claim 11, wherein the host organism is *Escherichia coli*.

13. A method of producing human immune interferon protein which comprises growing a transformant containing a recombinant DNA having a structural gene of human immune interferon downstream from a rec A promoter or promoters and recovering the human immune interferon protein accumulated in the culture broth.

14. A method according to Claim 13, wherein the transformant is *Escherichia coli*.

 

Figure 1

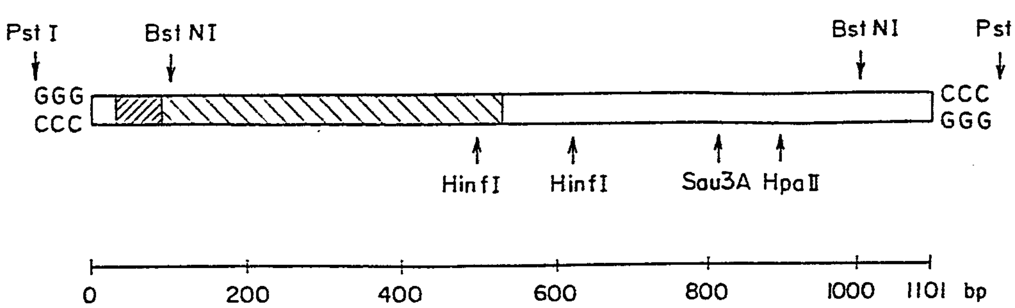
Pst I
Bst NI
Bst NI
Pst I
GGG
CCC
CCC
GGG
Hinf I
Hinf I
Sau3A
Hpa II
0
200
400
600
800
1000
1101 bp

Figure 2

```
                                   S1
5' ACTTCTTTGGCTTAATTCTCTCGGAAACG ATG AAA TAT ACA AGT TAT ATC TTG

                                                  S20  1
GCT TTT CAG CTC TGC ATC GTT TTG GGT TCT CTT GGC TGT TAC TGC CAG

                                                                20
GAC CCA TAT GTA AAA GAA GCA GAA AAC CTT AAG AAA TAT TTT AAT GCA

GGT CAT TCA GAT GTA GCG GAT AAT GGA ACT CTT TTC TTA GGC ATT TTG

            40
AAG AAT TGG AAA GAG CAG AGT GAC AGA AAA ATA ATG CAG AGC CAA ATT

                                60
GTC TCC TTT TAC TTC AAA CTT TTT AAA AAC TTT AAA GAT GAC CAG AGC

                                                  80
ATC CAA AAG AGT GTG GAG ACC ATC AAG GAA GAC ATG AAT GTC AAG TTT

                                                               100
TTC AAT AGC AAC AAA AAG AAA CGA GAT GAC TTC GAA AAG CTG ACT AAT

TAT TCG GTA ACT GAC TTG AAT GTC CAA CGC AAA GCA ATA CAT GAA CTC

            120
ATC CAA GTG ATG GCT GAA CTG TCG CCA GCA GCT AAA ACA GGG AAG CGA

                                140                       146
AAA AGG AGT CAG ATG CTG TTT CGA GGT CGA AGA GCA TCC CAG TAA TGG

TTGTCCTGCCTGCAATATTTGAATTTTAAATCTAAATCTATTTATTAATATTTAACATTATTT
ATATGGGGAATATATTTTTAGACTCATCAATCAAATAAGTATTTATAATAGCAACTTTTGTGT
AATGAAAATGAATATCTATTAATATATGTATTATTTATAATTCCTATATCCTGTGACTGTCTC
ACTTAATCCTTTGTTTTCTGACTAATTAGGCAAGGCTATGTGATTACAAGGCTTTATCTCAGG
GGCCAACTAGGCAGCCAACCTAAGCAAGATCCCATGGGTTGTGTGTTTATTTCACTTGATGAT
ACAATGAACACTTATAAGTGAAGTGATACTATCCAGTTACTGCCGGTTTGAAAATATGCCTGC
AATCTGAGCCAGTGCTTTAATGGCATGTCAGACAGAACTTGAATGTGTCAGGTGACCCTGATG
AAAACATAGCATCTCAGGAGATTTCATGCCTGGTGCTTCCAAATATTGTTGACAACTGTGACT
GTACCCAAATGGAAAGTAACTCATTTGTTAAAATTATCAATATCTAATATATATGAATAAAGT
G 3'
```

Figure 3

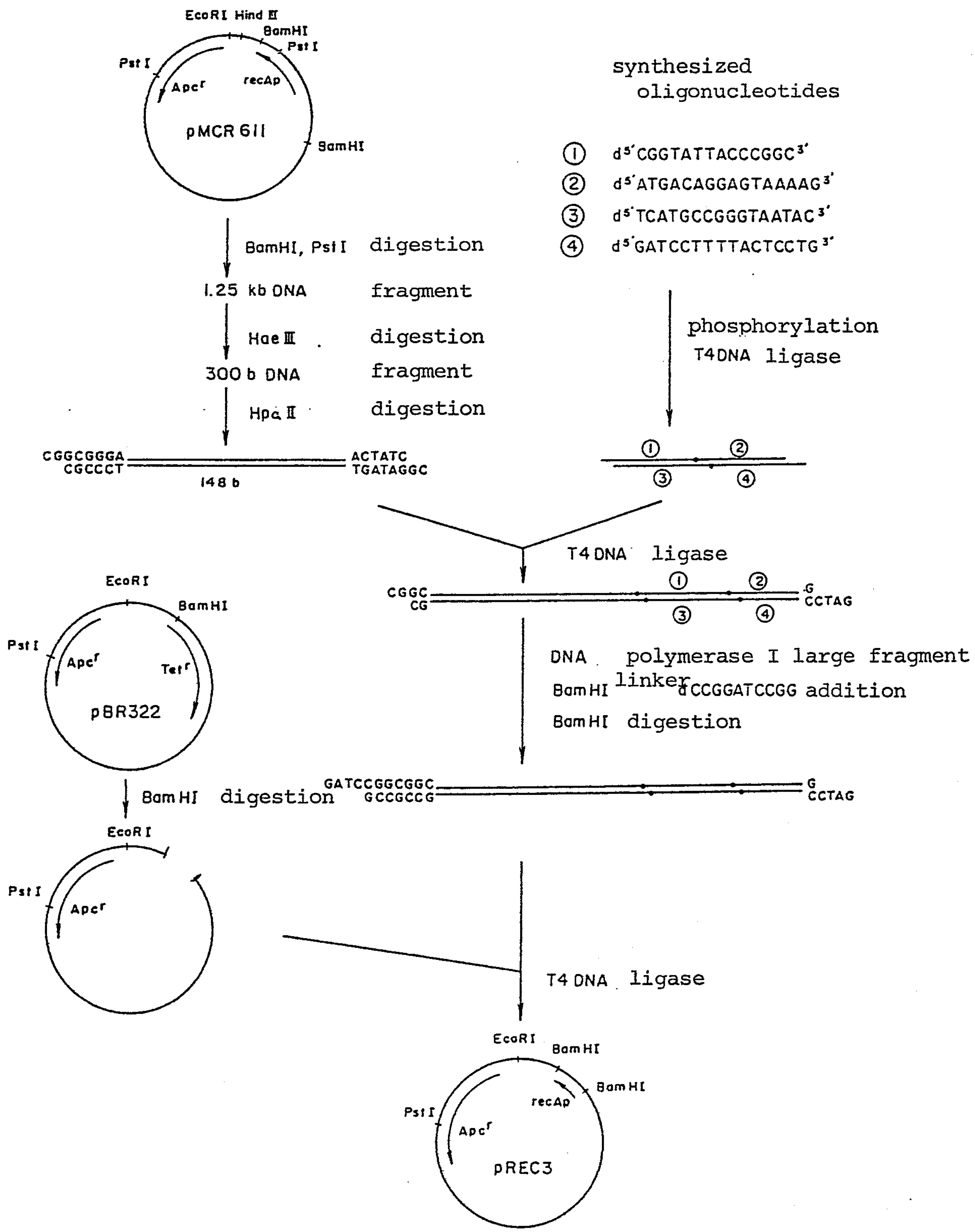
EcoRI Hind III
BamHI
PstI
PstI
Apc^r
recAp
pMCR 611
BamHI
synthesized oligonucleotides
① d5'CGGTATTACCCGGC3'
② d5'ATGACAGGAGTAAAAG3'
③ d5'TCATGCCGGGTAATAC3'
④ d5'GATCCTTTTACTCCTG3'
BamHI, PstI digestion
1.25 kb DNA fragment
HaeIII digestion
300 b DNA fragment
HpaII digestion
phosphorylation
T4DNA ligase
CGGCGGGA
CGCCCT
148 b
ACTATC
TGATAGGC
T4 DNA ligase
EcoRI
BamHI
PstI
Apc^r
Tet^r
pBR322
CGGC
CG
G
CCTAG
DNA polymerase I large fragment
BamHI linker dCCGGATCCGG addition
BamHI digestion
BamHI digestion
GATCCGGCGGC
GCCGCCG
G
CCTAG
EcoRI
PstI
Apc^r
T4 DNA ligase
EcoRI
BamHI
BamHI
PstI
recAp
Apc^r
pREC3

Figure 4

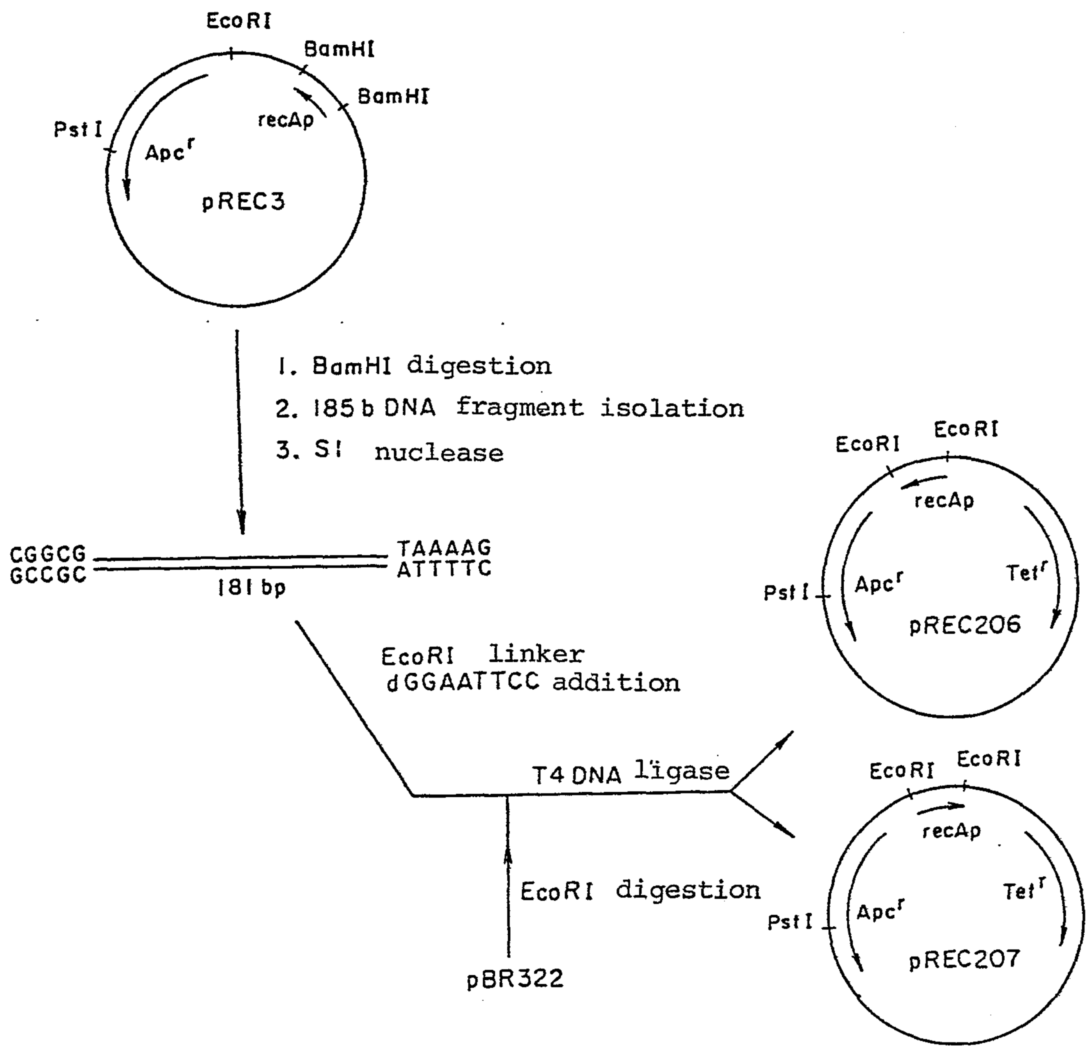
EcoRI
BamHI
BamHI
PstI
recAp
Apc^r
pREC3
1. BamHI digestion
2. 185 b DNA fragment isolation
3. S1 nuclease
CGGCG
GCCGC
TAAAAG
ATTTTC
181 bp
EcoRI linker
dGGAATTCC addition
T4 DNA ligase
EcoRI digestion
pBR322
EcoRI
EcoRI
recAp
PstI
Apc^r
Tet^r
pREC206
EcoRI
EcoRI
recAp
PstI
Apc^r
Tet^r
pREC207

Figure 5

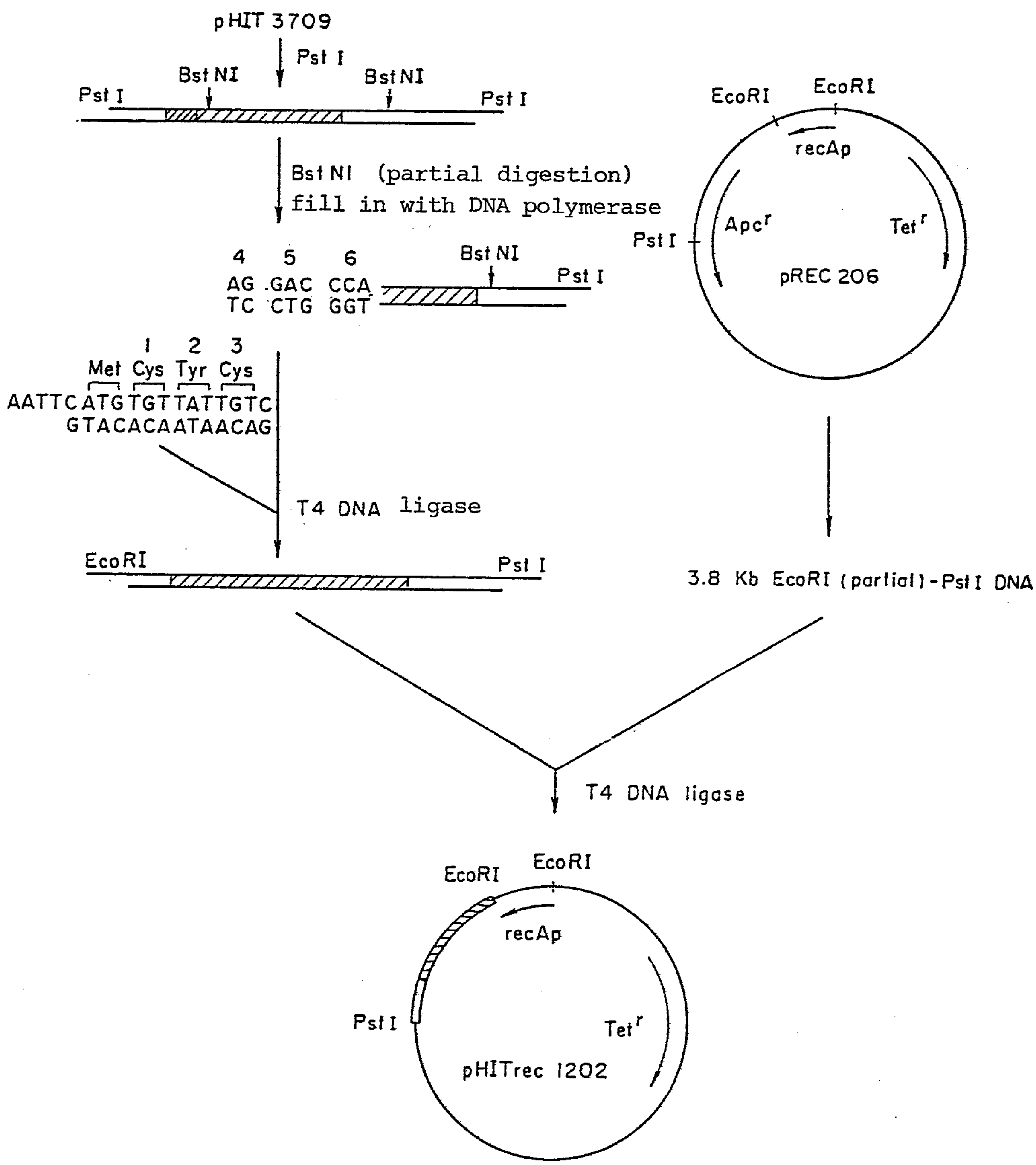
pHIT 3709
Pst I
Bst NI
Bst NI
Pst I
Pst I
Bst NI (partial digestion)
fill in with DNA polymerase
4 5 6
AG GAC CCA
TC CTG GGT
Bst NI
Pst I
1 2 3
Met Cys Tyr Cys
AATTCATGTGTTATTGTC
GTACACAATAACAG
T4 DNA ligase
EcoRI
Pst I
EcoRI
EcoRI
recAp
Apc^r
Tet^r
Pst I
pREC 206
3.8 Kb EcoRI (partial)-Pst I DNA
T4 DNA ligase
EcoRI
EcoRI
recAp
Pst I
Tet^r
pHITrec 1202

Figure 6

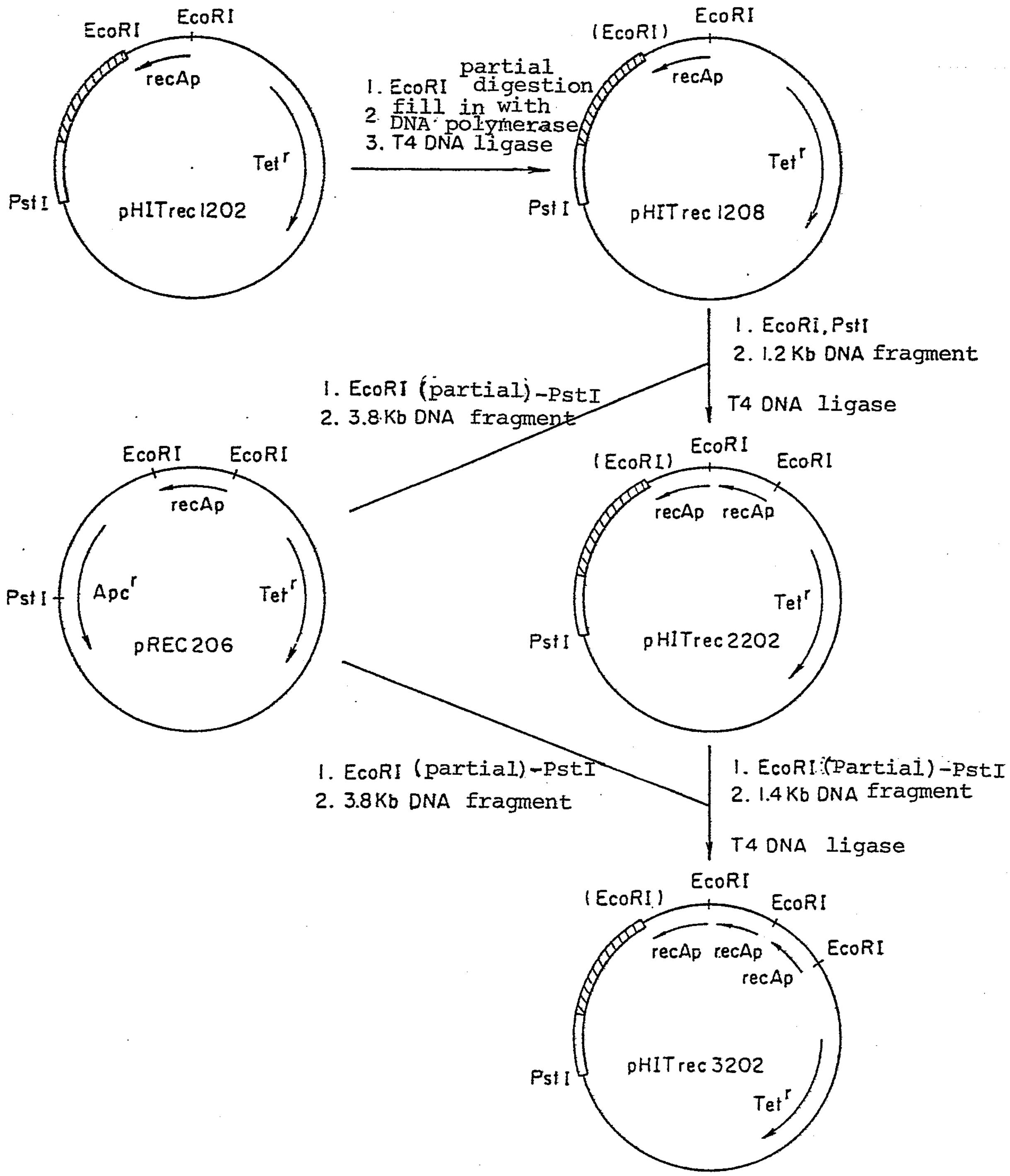
EcoRI
EcoRI
recAp
Tet^r
PstI
pHITrec1202
1. EcoRI partial digestion
2. fill in with DNA polymerase
3. T4 DNA ligase
(EcoRI)
EcoRI
recAp
Tet^r
PstI
pHITrec1208
1. EcoRI, PstI
2. 1.2 Kb DNA fragment
T4 DNA ligase
1. EcoRI (partial)-PstI
2. 3.8 Kb DNA fragment
EcoRI
EcoRI
recAp
Apc^r
Tet^r
PstI
pREC206
(EcoRI)
EcoRI
EcoRI
recAp
recAp
Tet^r
PstI
pHITrec2202
1. EcoRI (partial)-PstI
2. 3.8Kb DNA fragment
1. EcoRI (Partial)-PstI
2. 1.4 Kb DNA fragment
T4 DNA ligase
(EcoRI)
EcoRI
EcoRI
EcoRI
recAp
recAp
recAp
PstI
pHITrec3202
Tet^r

European Patent Office

# EUROPEAN SEARCH REPORT

0126230 Application number

EP 84 10 2775

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP-A-0 068 719 (TAKEDA) * Claims 1-26; pages 8,9; figure 7 * --- | 1,7 | C 12 N 15/00<br>C 12 N 1/20 //<br>(C 12 N 1/20<br>C 12 R 1/19 ) |
| D,Y | NATURE, vol. 295, 11th February 1982, pages 503-508, MacMillan Journals Ltd.; P.W. GRAY et al.: "Expression of human immune interferon cDNA in E. coli and monkey cells" * Whole document * --- | 2,8 | |
| D,Y | NUCLEIC ACIDS RESEARCH, vol. 10, no. 8, 1982, pages 2487-2501, IRL Press, GB; R. DEVOS et al.: "Molecular cloning of human immune interferon cDNA and its expression in eukaryotic cells" * Whole document * --- | 2,6,8 | TECHNICAL FIELDS SEARCHED (Int. Cl. 3) |
| D,Y | NUCLEIC ACIDS RESEARCH, vol. 10, no. 12, pages 3605-3615, IRL Press, GB; R. DERYNCK et al.: "Human interferon gamma is encoded by a single class of mRNA" * Discussion, page 6312 * --- -/- | 2,8 | C 12 N |

The present search report has been drawn up for all claims

| Place of search | Date of completion of the search | Examiner |
|---|---|---|
| THE HAGUE | 04-07-1984 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0126230

Application number

EP 84 10 2775



**DOCUMENTS CONSIDERED TO BE RELEVANT**

| Category | Citation of document with indication, where appropriate, of relevant passages | Relevant to claim | CLASSIFICATION OF THE APPLICATION (Int. Cl. 3) |
|---|---|---|---|
| D,Y | NUCLEIC ACIDS RESEARCH, Symposium Series no. 11, 1982, pages 29-32, IRL Press Limited, Oxford, GB; S. TANAKA et al.: "Expression in Escherichia coli of chemically synthesized gene for a human immune interferon" * Whole document * --- | 2,6,8 | |
| P,Y | NUCLEIC ACIDS RESEARCH, vol. 11, no. 9, May 1983, pages 2927-2941, Oxford, GB; S.I. FEINSTEIN et al.: "Expression of human interferon genes using the recA promoter of Escherichia coli" * Whole document * --- | 1,7 | |
| P,Y | EP-A-0 089 676 (TAKEDA) * Claims 1-20 * ----- | 1,6 | TECHNICAL FIELDS SEARCHED (Int. Cl. 3) |

The present search report has been drawn up for all claims

| Place of search | Date of completion of the search | Examiner |
|---|---|---|
| THE HAGUE | 04-07-1984 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document